# EUROPEAN PATENT SPECIFICATION

(11) **EP 2 596 376 B1**
(45) Date of publication and mention of the grant of the patent: **03.05.2017**
(21) Application number: 11752639.2
(22) Date of filing: 12.07.2011
(51) Int. Cl.: G01R 33/567, A61B 5/00, A61B 5/113, A61B 5/055, A61B 5/0205, G01R 33/28, A61B 5/021

(54) **DUAL PRESSURE SENSOR SIGNAL CHAIN TO REMOVE MUTUALLY-COUPLED MRI INTERFERENCE**
DOPPELDRUCKSENSORSIGNALKETTE ZUR BESEITIGUNG VON WECHSELSEITIG GEKOPPELTER MRT-INTERFERENZ
CHAÎNE DE SIGNAL DE CAPTEUR DE PRESSION DOUBLE POUR RETIRER L'INTERFÉRENCE IRM MUTUELLEMENT COUPLÉE

(30) Priority: 23.07.2010 US 366988 P
(43) Date of publication of application: 29.05.2013
(73) Proprietor: Koninklijke Philips N.V., 5656 AE Eindhoven (NL)
(72) Inventor: REY, Eduardo Mario, NL-5656 AE Eindhoven (NL); CATES, Harold Joseph, NL-5656 AE Eindhoven (NL)
(74) Representative: van Velzen, Maaike Mathilde
(86) International application number: PCT/IB2011/053093
(87) International publication number: WO 2012/011019

(56) References cited:
- WO-A2-2007/134144
- US-A- 5 209 233
- US-A1- 2003 018 248
- US-A1- 2007 172 029
- US-A1- 2008 269 581
- MICHAEL K LAUDON ET AL: "Minimizing Interference from Magnetic Resonance Imagers During Electrocardiography", IEEE TRANSACTIONS ON BIOMEDICAL ENGINEERING, IEEE SERVICE CENTER, PISCATAWAY, NJ, USA, vol. 45, no. 2, 1 February 1998 (1998-02-01), XP011006497, ISSN: 0018-9294

## Description

The following relates to the medical arts, magnetic resonance arts, physiological monitoring arts, and related arts. It finds application in magnetic resonance imaging and other magnetic resonance applications that are beneficially monitored by electrocardiography, and the like.

When a patient is undergoing a magnetic resonance imaging (MRI) scan procedure, the patient is position in a static magnetic field (B₀), e.g., inside the bore of the MRI scanner. Radiofrequency fields (B₁), and gradient magnetic field pulses (Gₓ, G_{y}, G_{z}) are applied and directed at the patient to induce resonance, spatially localize resonance signals, and the like. Electro medical devices inside or near the MRI bore, such as patient monitors and life support devices, are exposed to these types of fields. The B₀ field acts as a strong electromagnet that attracts ferromagnetic objects towards the magnet. The B₀ field is typically present as long as the magnet is powered (even when scans are not being performed). In addition, the B₁ field is generated by an RF coil that emits radiofrequency waves into free space at frequencies related by the Larmor equation. These frequencies are, for example, approximately 64 MHz and 128 MHz for 1.5T and 3.0T magnets, respectively. The gradient fields are generated by gradient coils that momentarily alter the static magnetic field (B₀) to produce a momentary change in magnetic field strength. Unlike the static field (B₀), the radio frequency (RF) field (B₁) and the MRI gradients (Gₓ, G_{y}, G_{z}) are generated and used only during actual scan sequences. The combination of these three fields allow for image reconstruction.

MR-compatible products must operate within specifications in the presence of these interference sources. To avoid this interference, respiration monitors, for example, commonly include a bladder, which is mounted to or around a patient's waist within the fields. A pneumatic tube connects the bladder with appropriate electronics mounted away from the scanner, e.g., beyond the 5 gauss line, outside the shielded room, or the like. Other monitors use light, such as lasers, video cameras, or fiber optics, to sense physiological status and convey the monitored status to remote electronics.

Laudon et. al., "Minimizing Interference from Magnetic Resonance Imagers during Electrocardiography", IEEE Transactions on Biomedical Engineering vol. 45 no. 2, 1 February 1998, describes a technique for removing induced MRI gradient interference from an ECG recorded on a patient inside the bore of a MRI scanner, For this purpose, a first sensor is located to sense the physiological status and experience MR scan related interference and to output a first signal having a physiological status component and an interference component. A second, non-active sensor is located closely adjacent to the first sensor to experience substantially the same MR scan related interference as the first sensor and output a second signal having an interference component. A circuit subtractively operates on the first and second signals to cancel the interference component. US 2007/172029 describes a motion monitor system for use with imaging systems.

The following provides a new and improved apparatus and methods which overcome the challenges discussed above and others.

In accordance with one aspect, a physiological status sensing device senses a physiological status of a patient and minimizes an amount of interference generated during a magnetic resonance scan. The device includes a first, active sensor which is located such that it can sense the physiological status of the patient, and will experience the MR scan related interference during an MR scan, and arranged to output a first signal during the scan having a physiological status component and an interference component. A second non-active sensor is located closely adjacent to the first sensor such that it will experience substantially the same MR related interference as the first sensor during an MR scan and arranged to output a second signal during the scan having the interference component. A circuit subtractively operates on the first and second signals to cancel the interference component. The first, active sensor includes a first pressure transducer arranged to sense a pressure related to the physiological status and the second, non-active sensor includes a second pressure transducer arranged so as to be prevented from sensing pressure.

In accordance with another aspect, a method for sensing the physiological status of a patient and minimizing an amount of interference generated during a magnetic resonance scan is provided. The physiological status and magnetic scan related interference are sensed by a first, active sensor to generate a first signal having a physiological status component and an interference component. The magnetic resonance scan related interference is also sensed by a second, non-active sensor positioned closely adjacent the first sensor such that it experiences substantially the same MR scan related interference as the first sensor to generate a second signal having the interference component. The first and second signals are subtractively combined to cancel the interference component and generate a signal with the physiological status component.
The first, active sensor includes a first pressure transducer arranged to sense a pressure related to the physiological status and the second, non-active sensor includes a second pressure transducer arranged so as to be prevented from sensing pressure.

One advantage resides in enabling sensor electronics to be placed in high field regions.

Another advantage resides in artifact-free or reduced physiological sensor signals.

Another advantage resides in compact size and elimination of pneumatic cabling..

Further advantages of the present invention will be appreciated to those of ordinary skill in the art upon reading and understand the following detailed description.
FIGURE 1 diagrammatically illustrates a magnetic resonance data acquisition system.
FIGURE 2 diagrammatically illustrates one embodiment of a respiratory sensor for use in the system of FIGURE 1 in combination with an interference filter for removing interference.
FIGURE 3 diagrammatically illustrates one embodiment of a sensor for use in the system of FIGURE 1 in combination with an interference filter for removing interference.
FIGURE 4 diagrammatically illustrates one embodiment of a sensor for use in the system of FIGURE 1 in combination with an interference filter for removing interference.
FIGURE 5 illustrates a method of monitoring physiological status of a patient in an MR environment.

Gradient fields (Gₓ, G_{y}, G_{z}), in particular, are a primary source of MRI interference in other electro-medical devices operating inside or near an MRI bore. In a three-dimensional space, when an MR imaging gradient is introduced, this appears as a fast or pulsed change in field strength that is superimposed across the patient to allow spatial localization. During this time, MRI gradient interference is inductively coupled into electro-medical devices (via loop or dipole-antenna effects) and can appear as differential-mode (DM) or common-mode (CM) interference. Therefore, the MRI gradients generated by the gradient field magnet are a primary interference source and the electronics on the Printed Circuit Assembly (PCA) are the victim. Because the MRI system uses a highly homogenous static field and the MRI gradients appear as wavefronts across the patient or a specific part of the patient, any two locations, near or inside the bore, exposed to the gradient field pulses that are relatively close to each other and oriented in the same direction, will be exposed to similar or highly correlated electromagnetic (EM) fields. These similar electromagnetic fields are referred to herein as "mutually-coupled electromagnetic interference." With this presumption of nearby devices being exposed to similar gradient fields, one embodiment of the present disclosure utilizes two pressure sensors mounted adjacent to each other and coupled to a signal chain architecture, to remove the electromagnetic interference that is mutually-coupled to both sensors.

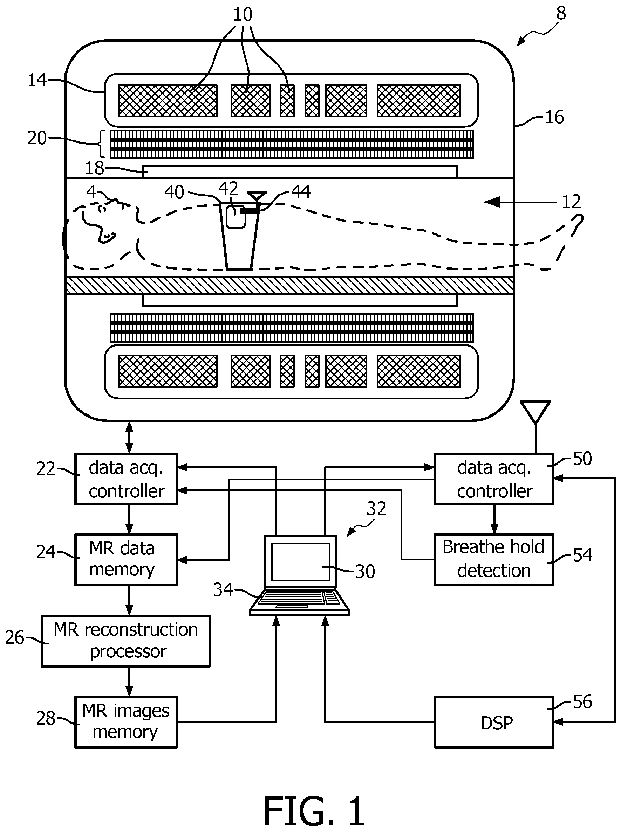

With reference to FIGURE 1, a magnetic resonance (MR) system includes a MR scanner **8** having a main magnet **10** that generates a static main (B₀) magnetic field in an examination region **12.** In the illustrated embodiment, the main magnet **10** is a superconducting magnet disposed in a cryogenic vessel **14** employing helium or another cyrogenic fluid; alternatively a resistive main magnet can be used. In the illustrated embodiment, the magnet assembly **10**, **14** is disposed in a generally cylindrical scanner housing **16** defining the examination region as a bore **12**, such as a cylindrical bore; alternatively, other geometries such as an open or other MR geometry can also be used. Magnetic resonance is excited and detected by one or more radio frequency coils (B₁), such as an illustrated whole-body quadrature body coil **18** or one or more local coils or coil arrays such as a head coil or chest coil. The excited magnetic resonance is spatially encoded, phase- and/or frequency-shifted, or otherwise manipulated by magnetic field gradients selectively generated by a set of magnetic field gradient coils **20**.

The magnetic resonance scanner **8** is operated by a magnetic resonance data acquisition controller **22**, suitably embodied by a dedicated digital processing device, a suitably programmed general purpose computer, or so forth, to generate, spatially encode, and read out magnetic resonance data, such as projections or k-space samples, that are stored in a magnetic resonance data memory **24**. The acquired spatially encoded magnetic resonance data are reconstructed by a magnetic resonance reconstruction processor **26** to generate one or more images of a patient or subject **4** disposed in the examination region **12**. The reconstruction processor **26** employs a reconstruction algorithm comporting with the spatial encoding, such as a backprojection-based algorithm for reconstructing acquired projection data, or a Fourier transform-based algorithm for reconstructing k-space samples. The one or more reconstructed images are stored in a magnetic resonance images memory **28**, and are suitably displayed on a display **30** of a computer system **32**, or printed using a printer or other marking engine, or transmitted via the Internet or a digital hospital network, or stored on a magnetic disk or other archival storage, or otherwise utilized. The illustrated computer sytem **32** also includes one or more user input devices such as an illustrated keyboard **34**, or a mouse or other pointing-type input device, or so forth, which enables a radiologist, or other clinician user to manipulate images and, in the illustrated embodiment, interface with the magnetic resonance scanner controller **22**.

With continuing reference to FIGURE 1, the MR system includes a physiological status sensing device **40**, in the form of a pressure sensing device that includes one or more pressure transducers **42** operatively connected to the computer system **32.** The pressure transducer(s) may be be included in a respiratory sensor, an invasive or non-invasive blood pressure sensor, or any other medical device operative to utilize pressure, light, or other non-electrical medium for acquring patient data inside the bore **12** of the MR system. In one embodiment the physiological status sensing device **40** includes a belt or cuff **44** that extends around a patient's torso or a limb. The physiological status sensing device **40** senses pressure stimuli within the bore **12** and then wirelessly sends data to a monitoring signal acquisition device **50**. The physiological status sensing device **40** further includes a wireless transmitter (not shown) for sending wireless pressure signals to the monitoring signal acquisition device **50**. The present disclosure is not limited to a wireless telemetry and may also include a fiber optic, body coupled near field, wired solutions, or the like.

The monitor signal acquisition device **50**, in one embodiment, acquires pressure signals from the physiological status sensing device **40**, determines a respiratory or other physiological state, and stores the physiological state with the MR data in the MR data memory **24**. This physiological state information can be used for example in retrospective gating to sort the data by physiological state. In this manner, the MR data can be reconstructed based on respiratory or other physiological state.

In another embodiment, a breath hold detection circuit **54** detects the patient's breathe hold status and communicates the breathhold status to the MR data acquisition controller **22**. The MR data acquisition controller can use the breathhold status for prospective gating to limit data acquisition to a breathhold or other respiratory state. A digital signal processor **56** is also included for processing the physiological status signals, for example, to generate a respiratory cycle display on the monitor **30**. The present disclosure is not limited to respiratory monitoring, but is operable with any medical device for sensing pneumatic or other stimulus and processing the signals therefrom.

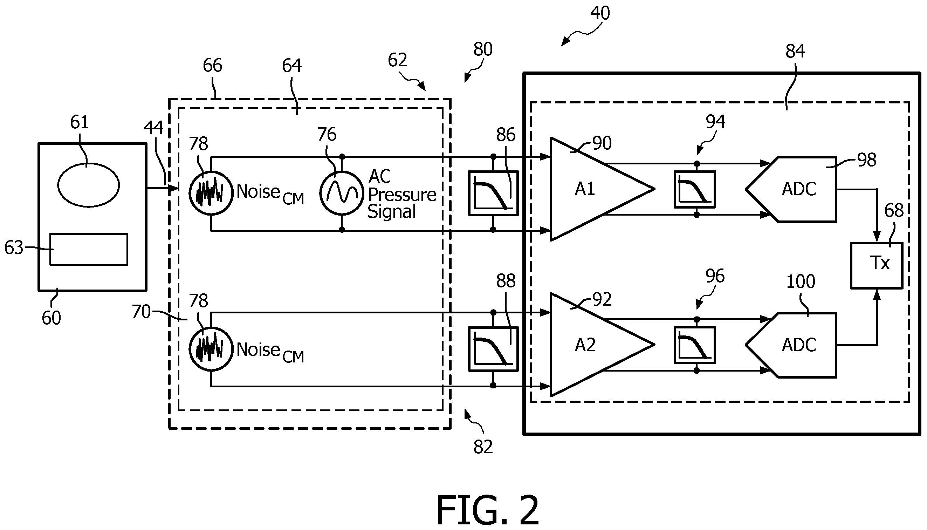

With reference to FIGURE 2, illustrates an exemplary embodiment of the physiological status sensing device **40** having a patient interface **60**, a signal processing chain **62** coupled thereto, and a transmitter **68**. The patient interface **60** comprises an invasive or non-invasive measuring element, for example, for a blood pressure module such as a blood pressure dome **63**, or a pneumatic respiratory measurement system such as a bladder **61**. In the respiratory example, a belt or strap **44** extends around the patient's chest. As the patient's chest expands and contracts during breathing, the pressure in the bladder increases and decreases. A first or active pressure to electrical signal transducer **64**, such as a piezoresistive sensor, converts the breathing related pressure variations into electrical signals.

The first or active sensor **64** is mounted on a sensor module **66** with a second or dummy sensor **70**. The first sensor **64** and second sensor **70** are substantially identical pressure-to-electric converters, such as piezoesistive pressure transducers located closely adjacent to one another in a same direction and a same axis so that each sensor is exposed to a same amount of interferene, such as a same magnetic gradients (Gₓ, G_{y}, G_{z}) having substantially the same amplitudes and phases with one another. While both sensors are mounted adjacent to each other, only the first is active and actually connected to the bladder pressure dome to monitor pressure changes. The other second is a non-active (i.e., a dummy) sensor which is mounted so that it does not sense the pressure changes. In this manner, this first, active sensor **64** generates an output electrical signal which is the sum of a pressure component **76** indicative of pressure changes and a noise signal **78** indicative of the magnetic gradient interference, which is generated during actual scanning processes. The second, inactive sensor **70** outputs the same noise signal **78**, but has no presssure component.

In one embodiment, the first sensor **64** and the second sensor **70** comprise piezoresistive Wheatstone bridge circuits, which can be utilized for invasive pressure monitoring applications, for example. Wheatstone bridge circuits have a differential-mode voltage ouput that is proportional to a change in pressure. In this embodiment, the first sensor **64** and the second sensor **70** generate a first differential ouput **80** and a second differential ouput **82**, respectively, with each having a differential-mode output voltage. Typically, Wheatstone bridge circuits are constructed from four resistors, one of which has a calibration value (Rₓ), one of which is variable (R**₂**), e.g. with changes in pressure, and two of which are fixed and equal (R**₁** and R**₃**), connected as the sides of a square. Two opposite corners of the square are connected to a source of electrical current, such as a battery. The other two opposite corners output the differential output. Wheatstone bridge circuits are well known by one of ordinary skill in the art and provide many applications with principles that are not therefore explained in great detail herein.

The first differential output **80** and second differential output **82** from the first and second sensors, respectively, are coupled to an interference filter **84** with a first differential mode and common mode low pass filter **86** and a second differential mode and common mode low pass filter **88**, respectivley, coupled therebetween. The filters **86** and **88** comprise differential mode filters that filter any signal that is differential in nature, such as the AC signal **76** generated by the pressure stimuli and any noise **78** that is electromagnetically coupled to both sensors. Both of the signals **76** and **78** are differential in nature (i.e., transmitted with two complementary signals), with a spectral band width of zero to 10 hertz for the pressure stimulus signal **76** and the coupled noise signal **78** extending up to about 30 kilohertz, for example. Consequentially, the differential mode filter serves to filter both of these signals. The electromagnetic interference components above a cutoff frequency above the frequency of the pressure signals, e.g. about 1 kilohertz, for example, are removed. This leaves the part of the noise signal that is in the same bandwidth as the pressure signal. The common mode part of the first and second filters addresses noise from pathways and devices of both signal paths (e.g., the sensor devices and differential ouput connections) in addition to any external interference.

With continued reference to FIGURE 2, the interference filter **84** includes a first instrument amplifier **90** and a second interference amplifier **92** respectively coupled to the first differential output **80** and the second differential ouput **82**. The first and second instrument amplifiers **90**, **92** each include a differential amplifier, which is a type of electronic device that multiplies the difference between two inputs by a constant factor or differential gain. A third and fourth differential mode and common mode low pass filter **94**, **96** further filters the differential ouputs of respective amplifiers **90** and **92** before analog signals including the noise and AC pressure signal are convertered at converters **98** and **100**. The converters **98** and **100** include fully differential analog to digital converters for converting analog signals to digital signals. These digital signals are afterwards either transmitted by the radio frequency transmitter **68** wirelessly at a frequency that does interfere with the MR system scanning. Alternatively, the digital signals are sent out on an optic fiber, or other communication link (not shown). The DSP **56** discussed in conjunction with FIGURE 1, in one embodiment, fiters the digital signal to remove the remaining noise components of the signal by appropriate algorithms so that the pressure signal is locally and/or remotely displayed, for example, at display **30**. For example, the signal from converter **98** has a pressure signal component and a noise component and the signal from converter **100** has only the noise component. Because both noise components are the same, subtractively combining the signals leaves the pressure signal component. Consequently, two differential digital signals produced as output to the converters **98** and **100** are transmitted out of the bore and to a remote signal processing area. The DSP **56** could be located inside or outside the bore **12** of the MR system. MR imaging gradient artifacts and other interfering signals are thus effectively rejected and the stimulus pressure waveform is captured as a strong, clean signal.

The wiring of the pressure sensing device preferable has a length less than 5 cm to minimize a potential for the B₁ fields including current therein.

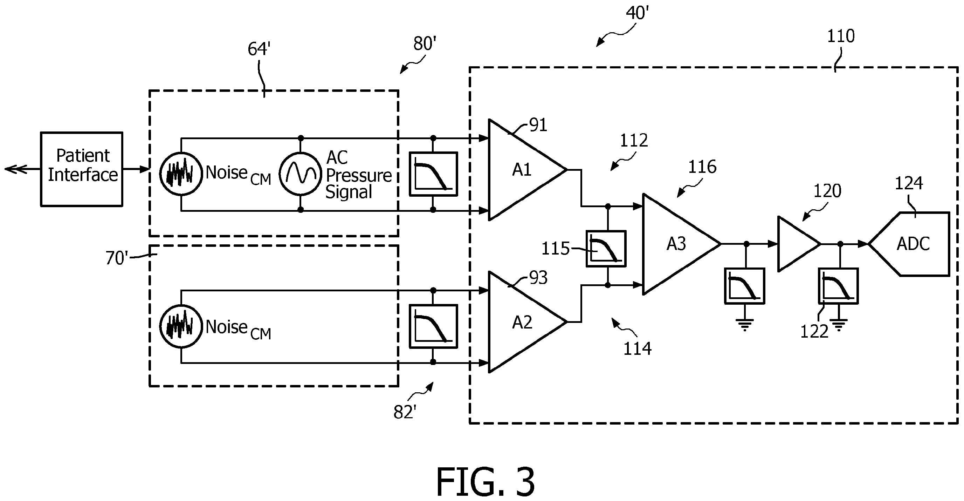

With reference to FIGURE 3, another embodiment of a pressure sensing device **40'** for minimizing an amount of interference generated during an MR scan in an MR system is illustrated. The device **40'** includes a signal chain architecture operable as an interference filter **110** for filtering mutually-coupled MR imaging (MRI) interference. A first differential output **80'** and a second differential output **82'** from a pressure sensing transducer **64'** and a like dummy transducer **70'** shielded from sensing pressure are supplied to the interference filter **110.**

The interference filter **110** includes a first instrument amplifier 91 and a second instrument amplifier **93** having a third output **112** and a fourth output **114**, respectively. Each amplifier includes a differential amplifier that receives a differential-mode output voltage and is configured to convert the voltage to a single-ended voltage output at the third and fourth outputs respectively. Each single-ended voltage is therefore generated from the outputs of an active pressure transducer **64'and** a non-active pressure transducer **70'**.

A third amplifier **116** receives as an input each single-ended output voltage from the first and second amplifiers **91**, **93** after being filtered by a third filter **115**. The third amplifier **116** subtracts the common mode signals to cancel the noise that is common to both inputs while amplifying the differential signals via the high common mode reduction capability therein, e.g., around 95 db at a unity gain or gain of zero decibels. For example, the third amplifier **116** is an instrumentation amplifier that removes the common mode noise, amplifies the differential, and then transmits a clean pressure signal downstream to an ADC driver **120** with an ADC filter **122**, which decouples charge from the sampling nature of a converter **124**. The converter **124** includes an analog digital converter to convert the analog signals to digital format for further processing and transmission. Low pass filters are used to remove high frequency noise generated by the system and devices therein to achieve a deeper roll off of the pressure signal.

The digital signal generated from the converter **124** can be either transmitted by an RF transmitter (not shown) that does not interfere with the MR system, an optic fiber, or other communication link for digital processing. In addition, the digital signal could be transmitted from within the bore and to a local display or external interface for viewing.

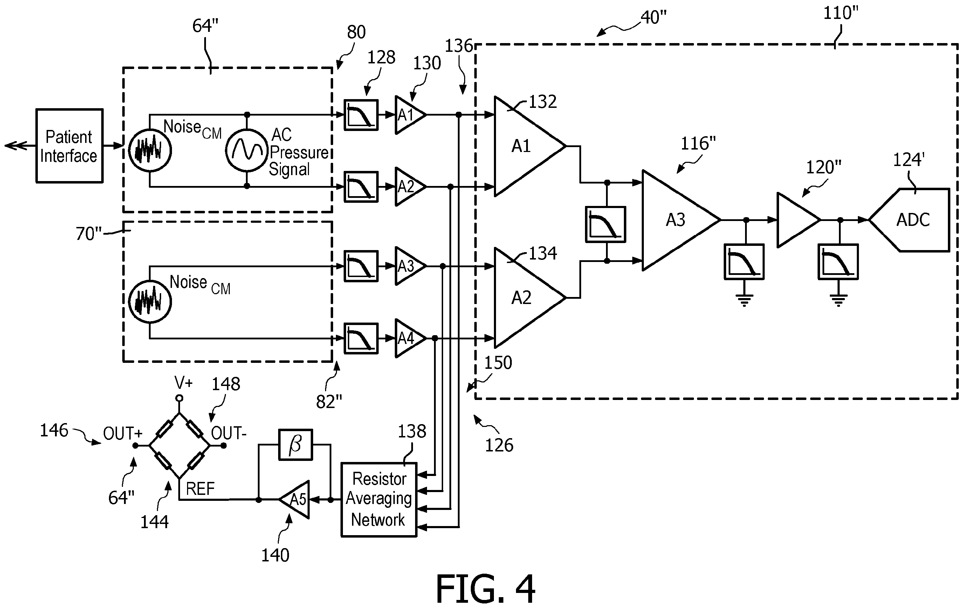

In FIGURE 4, like elements with FIGURES 2 and 3 are denoted by the same reference numeral followed by a double prime ("). An active drive stage **126** integrated into a pressure sensing device **40"** for further interference rejection is illustrated. Dual pressure sensors including an active pressure sensor **68"** and a non-active pressure sensor **70"** that is prevented from sensing pressure are electromagnetically coupled to one another to transmit a similar differential noise signal at four different output terminals. A first pair of output terminals define a positive and negative differential mode voltage terminal pair **80"** which carry an AC pressure signal captured by the first sensor and a noise component. A second pair of output terminals define a differential mode pair **82"** which carry the component. A low pass filter **128** filters higher frequency signals at each terminal, which is interfaced with the active drive stage **126**.

The active drive stage **126** includes high impedance buffers **130** that receive each positive and negative compliment of the differential signals before interfacing with a first amplifier **132** and a second amplifier **134**. The buffers **130** are single-ended buffers that respectively receive each plus or minus differential voltage generated by the sensors **64"** and **70"**. Each output of differential outputs **80"** and **82"** are processed independently and fed to the first and second amplifiers **132** and **134**, respectively. The outputs of amplifiers **132**, **134** are subtractively combined by amplifier **116"** and converted to a digital pressure signal by an analog-to-digital converter **124".** Consequently, the inputs to the instrument amplifiers **132** and **134** receive low impedance output signals, rather than a moderate impedance input source that they would receive if they were more directly interfaced to the pressure transducers **68"** and **70"** as illustrated in FIGURE 3. The outputs of the buffers **130** are also connected with a tap **150** for directing the high impedance signal to a resistor averaging network **138.**

The high impedance output signals are averaged by the resistor averaging network **138** where each signal that is common to all the outputs of the buffers **130** is averaged with the other signals and outputted to an inverter **140** having a gain β device in parallel providing a gain factor β to the signal. The signal is further provided to a reference voltage terminal **144** to the active pressure sensor **68"**, which is illustrated as a Wheatstone bridge circuit with a reference voltage terminal **144** and a plus and minus differential voltage output terminals **146**, **148** of the differential mode voltage output terminal **80".** The same reference signal from the inverter **140** can be fed to the reference voltage terminal of the Wheatstone bridge of the dummy pressure transducer **70".**

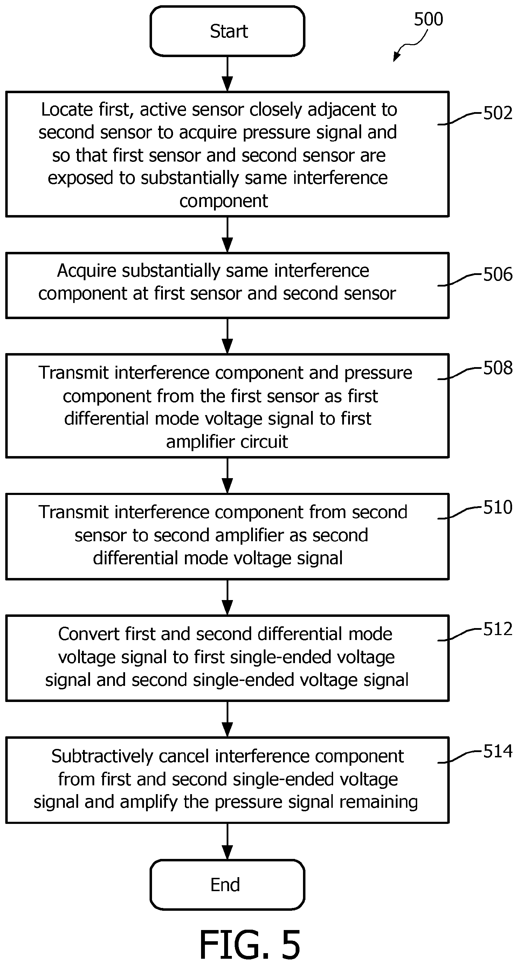

An example methodology **500** for removing interference from within an MRI bore during scanning is illustrated in FIGURE 5. While the method **500** is illustrated and described below as a series of acts or events, it will be appreciated that the illustrated ordering of such acts or events are not to be interpreted in a limiting sense. For example, some acts may occur in different orders and/or concurrently with other acts or events apart from those illustrated and/or described herein. In addition, not all illustrated acts may be required to implement one or more aspects or embodiments of the description herein. Further, one or more of the acts depicted herein may be carried out in one or more separate acts and/or phases.

The method **500** initializes at start and at **502** a pressure signal is acquired by locating a first, active sensor. In one embodiment, the first sensor is located closely adjacent to the second, dummy sensor so that the first sensor and second sensor are exposed to an interference that is substantially equal or the same in amplitude and phase. In one embodiment, two sensors are piezoresistive Wheatstone bridge circuits that are intended for invasive pressure monitoring applications. Physically, both pressure transducers are mounted closely adjacent to each other in the same direction and axis so that both sensors are exposed to similar levels of interference, such as the gradient pulse fields. While both sensors are mounted adjacent to each other, only one is actually receiving a pressure stimulus and the other is prevented from receiving a pressure stimulus. Both receive highly correlated electromagnetic interference. Wheatstone bridge circuits have a differential-mode voltage output that is proportional to a change in pressure. Since there are two sensors, there are two differential voltages at the input to the analog front-end. Therefore, the pressure signal together with interference is transmitted from the first sensor as a first differential mode voltage signal to a circuit having a first amplifier circuit at **508**, and the second sensor transmits a second differential mode voltage signal to a second amplifier at **510.**

In one embodiment, the first and second amplifiers are two high-speed instrumentation amplifiers that convert the differential-mode output voltage from the two pressure transducers into two single-ended voltages at **512**; alternatively, the outputs could be differential outputs if the amplifiers are differential amplifiers. In this stage, both amplifiers capture similar interfering fields, while only one also captures a pressure signal.

At **514** the interference that is common mode interference of both the first and second differential mode voltage signals is canceled by subtracting the interference component from the first and second signals. For example, the single ended mode voltage signals are operatively connected to a difference amplifier. Because the instrumentation amplifier is a difference amplifier, the next stage, another instrumentation amplifier, for example, takes the difference between these two signals which are: [Pressure Signal + Noise_{cm} ] and [Noise_{cm} ]. In another embodiment, the signals are digitized and digitally subtracted.

An advantage of the method is that it is effective in cancelling MRI gradient artifact in various applications that use a piezoresistive pressure transducer and is not limited to any one particular application or field of application. The pressure signal consequently can be wireless transmitted from within the bore of an MR system. A majority of interference is inductively coupled at the pressure transducer through Faraday's law of induction and effectively canceled.

The invention has been described with reference to the preferred embodiments. Modifications and alterations may occur to others upon reading and understanding the preceding detailed description. It is intended that the invention be construed as including all such modifications and alterations insofar as they come within the scope of the appended claims.

## Claims

1. A physiological status sensing device (40, 40', 40") for sensing a physiological status of a patient (4) and minimizing an amount of interference (78) generated during a magnetic resonance (MR) scan by a magnetic resonance (MR) system (8), the device (40) comprising:
a first, active sensor (64, 64', 64") located such that it can sense the physiological status and will experience MR scan related interference during an MR scan, and arranged to output a first signal (80, 80', 80") during the scan having a physiological status component (76) and an interference component (78);
a second, non-active sensor (70, 70', 70") located closely adjacent to the first sensor (64, 64', 64") such that it will experience substantially the same MR scan related interference as the first sensor (64, 64', 64") during an MR scan, and arranged to output a second signal (82, 82', 82") during the scan having the interference component (78); and
a circuit (50, 84, 110, 110', 116, 116') which subtractively operates on the first (80, 80', 80") and second signals (82) to cancel the interference component (78)
wherein the first, active sensor includes a first pressure transducer arranged to sense a pressure related to the physiological status and the second, non-active sensor includes a second pressure transducer arranged so as to be prevented from sensing pressure.

2. The device (40, 40', 40") according to claim 1, wherein the first and second pressure transducers comprise piezoresistive pressure transducers of like construction.

3. The device (40, 40', 40") according to either one of claims 1 and 2, wherein the first (64, 64', 64") and second sensors (70, 70', 70") have like construction and are mounted with a common orientation to a common substrate.

4. The device (40, 40', 40") according to any one of claims 1-3 further including:
an interference filter unit (110, 110', 110") that is electrically coupled to the first signal (80, 80', 80") having a first differential output (80, 80', 80") and the second signal (82, 82', 82") having a second differential output (82, 82', 82") of the first sensor (64, 64', 64") and the second sensor (70, 70', 70") respectively, and that is arranged to remove the interference component (78) from the first (80, 80', 80") and the second differential output (82, 82', 82"), and to transmit the physiological status component (76), wherein the first (80, 80', 80") and the second differential output (82, 82', 82") each have complementary output signals transmitted thereat.

5. The device (40, 40', 40") according to any one of claims 1-3 wherein the circuit (110, 110', 110") includes:
a first amplifier (91, 132) coupled to the first sensor (64, 64', 64") and having a third output (112);
a second amplifier (93, 134) coupled to the second sensor (70, 70', 70") and having a fourth output (114);
a differential amplifier (116, 116', 116") arranged to subtractively combine the third and fourth outputs to generate a pressure signal; and
a converter (124, 124', 124") arranged to digitize the pressure signal.

6. The device according to any one of claims 1-5, wherein at least one of the active sensor (64") and the non-active sensor (70") are in a Wheatstone bridge and further including:
an active driver stage (126) coupled to a reference terminal (144) of the first, active sensor (64") and including:
high impedance buffers (130) coupled to the first sensor (64") and the second non-active sensor (70") to provide a high impedance output (136) at each sensor output (80", 82");
a resistor averaging network (138) arranged to average each high impedance output (136) of the high impedance buffers (130) and to generate an averaged signal therefrom; and
a gain device (β) coupled to an inverter (140) arranged to invert the averaged signal and to bias the Wheatstone bridge with the inverted averaged signal.

7. The device (40, 40', 40") according to any one of claims 1-6, wherein the physiological status is a respiratory state and function, the device further including:
a belt or strap (44) configured to encircle the patient's waist;
a bladder (61) mounted to the belt (44) and arranged to be compressed during the patient's respiratory cycle, wherein the first sensor (64, 64', 64") is connected to the bladder to sense pressure therein.

8. The device (40, 40', 40") according to any one of claims 1-6, wherein the physiological status is a blood pressure state, the device further including:
a dome (63) mounted to be compressed with changes in blood pressure, wherein the first sensor (64, 64', 64") is connected to the dome (63) to sense pressure therein.

9. An MRI system comprising:
a main magnet (10) arranged to generate a static magnetic field in a patient;
gradient coils (20) arranged to impose gradient magnetic fields on the static magnetic field;
radio frequency coils (18) arranged to induce radio frequency fields;
a controller (22) arranged to control the gradient coils and the radiofrequency coils to acquire magnetic resonance information from the patient; and
the physiological status sensing device (40, 40', 40") according to any one of claims 1-8.

10. The MRI system according to claim 9, wherein the controller (22) is arranged to receive the output of the physiological status sensing device and to control the gradient and radiofrequency coils to acquire magnetic resonance information during a preselected physiological status of the patient.

11. The MRI system according to claim 9, further including:
a reconstruction processor (26) arranged to receive the output of the physiological status sensing device and to reconstruct images of the patient in one or more selected physiological status.

12. A method (500) for sensing a physiological status of a patient (4) and minimizing an amount of interference generated during a magnetic resonance (MR) scan, the method comprising:
with a first, active sensor (64, 64', 64") sensing the physiological status and MR scan related interference and generating a first signal (80, 80', 80") having a physiological status component (76) and an interference component (78);
with a second non-active sensor (70) positioned closely adjacent to the first sensor (64, 64', 64") such that it experiences substantially the same MR scan related interference as the first sensor (64, 64', 64") sensing the MR scan related interference and generating a second signal (82) having the interference component; and
subtractively combining the first and second signals to cancel the interference component (78) and generate a signal with the physiological status component;
wherein the first, active sensor includes a first pressure transducer arranged to sense a pressure related to the physiological status and the second, non-active sensor includes a second pressure transducer arranged so as to be prevented from sensing pressure.

13. The method according to claim 12, wherein the sensed physiological status varies in a predefined frequency range and further including:
filtering the first and second signals to remove frequency components above the predefined frequency range.

14. A method for operating an MRI system comprising:
generating static magnetic fields in a patient;
imposing gradient magnetic fields on the static magnetic fields;
imposing radio frequency fields to induce magnetic resonance in the patient;
acquiring magnetic resonance information from the patient;
sensing a physiological status of the patient with the method according to claim 12 or claim 13.

15. The MRI method according to claim 14, further including one of:
using the physiological status component to control imposing the gradient magnetic fields and the radio frequency fields so as to acquire the magnetic resonance information only when the patient has a selected physiological status; or
reconstructing the acquired magnetic resonance information into images and using the physiological status component to control the reconstructing so as to reconstruct the images from magnetic resonance information when the patient had one or more selected physiological status.

## Patentansprüche

1. Physiologische Zustandsmessvorrichtung (40, 40', 40") zum Messen eines physiologischen Zustands eines Patienten (4) und Minimieren eines Maßes an Interferenz (78), die während eines Magnetresonanz- (MR) Scans durch ein Magnetresonanz- (MR) System (8) erzeugt wird, wobei die Vorrichtung (40) Folgendes umfasst:
einen ersten aktiven Sensor (64, 64', 64"), der derartig angeordnet ist, dass er den physiologischen Zustand messen kann und während eines MR-Scans eine MR-Scan-bezogene Interferenz erfährt, und vorgesehen ist, um während des Scans ein erstes Signal (80, 80', 80") mit einer physiologischen Zustandskomponente (76) und einer Interferenzkomponente (78) auszugeben;
einen zweiten nicht-aktiven Sensor (70, 70' 70"), der eng angrenzend an den ersten Sensor (64, 64', 64") angeordnet ist, so dass er während eines MR-Scans im Wesentlichen die gleiche MR-bezogene Interferenz erfährt wie der erste Sensor (64, 64', 64"), und vorgesehen ist, um während des Scans ein zweites Signal (82, 82', 82") mit der Interferenzkomponente (78) auszugeben; und
eine Schaltung (50, 84, 110, 110', 116, 116'), die subtraktiv mit den ersten (80, 80', 80") und zweiten Signalen (82) arbeitet, um die Interferenzkomponente (78) aufzuheben,
wobei der erste aktive Sensor einen ersten Druckwandler umfasst, der dafür vorgesehen ist, einen Druck in Zusammenhang mit dem physiologischen Zustand zu messen, und wobei der zweite nicht-aktive Sensor einen zweiten Druckwandler umfasst, der so vorgesehen ist, dass er daran gehindert wird, Druck zu messen.

2. Vorrichtung (40, 40', 40") nach Anspruch 1, wobei der erste und der zweite Druckwandler piezoresistive Druckwandler von gleicher Bauart umfassen.

3. Vorrichtung (40, 40', 40") nach einem der Ansprüche 1 und 2, wobei die ersten (64, 64', 64") und zweiten Sensoren (70, 70', 70") von gleicher Bauart sind und mit der gleichen Ausrichtung an einem gemeinsamen Substrat montiert sind.

4. Vorrichtung (40, 40', 40") nach einem der Ansprüche 1 bis 3, weiterhin Folgendes umfassend:
eine Interferenzfiltereinheit (110, 110', 110"), die elektrisch mit dem ersten Signal (80, 80', 80") mit einem ersten differentiellen Ausgang (80, 80', 80") und dem zweiten Signal (82, 82', 82") mit einem zweiten differentiellen Ausgang (82, 82', 82") des ersten Sensors (64, 64', 64") bzw. des zweiten Sensors (70, 70', 70") gekoppelt ist, und die vorgesehen ist, um die Interferenzkomponente (78) aus dem ersten (80, 80', 80") und dem zweiten differentiellen Ausgang (82, 82', 82") zu entfernen und um die physiologische Zustandskomponente (76) zu übertragen, wobei an dem ersten (80, 80', 80") und dem zweiten differentiellen Ausgang (82, 82', 82") jeweils komplementäre Ausgangssignale gesendet werden.

5. Vorrichtung (40, 40', 40") nach einem der Ansprüche 1 bis 3, wobei die Schaltung (110, 110', 110") Folgendes umfasst:
einen ersten Verstärker (91, 132), der mit dem ersten Sensor (64, 64', 64") gekoppelt ist und einen dritten Ausgang (112) hat;
einen zweiten Verstärker (93, 134), der mit dem zweiten Sensor (70, 70', 70") gekoppelt ist und einen vierten Ausgang (114) hat;
einen differentiellen Verstärker (116, 116', 116"), der dafür vorgesehen ist, den dritten und den vierten Ausgang subtraktiv zu kombinieren, um ein Drucksignal zu erzeugen; und
einen Konvertierer (124, 124', 124"), der dafür vorgesehen ist, das Drucksignal zu digitalisieren.

6. Vorrichtung nach einem der Ansprüche 1 bis 5, wobei mindestens entweder der aktive Sensor (64") oder der nicht-aktive Sensor (70") in einer Wheatstone-Brücke vorliegt und weiterhin Folgendes umfasst:
eine aktive Treiberstufe (126), die mit einem Referenzanschluss (144) des ersten aktiven Sensors (64") gekoppelt ist und Folgendes umfasst:
hochohmige Puffer (130), die mit dem ersten Sensor (64") und dem zweiten nicht-aktiven Sensor (70") gekoppelt sind, um eine hochohmige Ausgabe (136) an jedem Sensorausgang (80", 82") bereitzustellen;
ein Widerstandsmittelwertbildungsnetzwerk (138), das dafür vorgesehen ist, für jede hochohmige Ausgabe (136) des hochohmigen Puffers (130) eine Mittelwertbildung durchzuführen und ein gemitteltes Signal daraus zu erzeugen; und
eine Verstärkungsvorrichtung (β), die mit einem Inverter (140) gekoppelt ist und vorgesehen ist, um das gemittelte Signal zu invertieren und die Wheatstone-Brücke mit dem invertierten gemittelten Signal vorzuspannen.

7. Vorrichtung (40, 40', 40") nach einem der Ansprüche 1 bis 6, wobei der physiologische Zustand ein Atemzustand und eine Atemfunktion ist, wobei die Vorrichtung weiterhin Folgendes umfasst:
einen Riemen oder Gurt (44), der konfiguriert ist, um die Taille des Patienten zu umgeben;
eine Blase (61), die an dem Riemen (44) montiert ist und vorgesehen ist, um während eines Atemzyklus des Patienten komprimiert zu werden, wobei der erste Sensor (64, 64', 64") mit der Blase verbunden ist, um den Druck darin zu messen.

8. Vorrichtung (40, 40', 40") nach einem der Ansprüche 1 bis 6, wobei der physiologische Zustand ein Blutdruckzustand ist, wobei die Vorrichtung weiterhin Folgendes umfasst:
eine Wölbung (63), die montiert ist, um mit Änderungen im Blutdruck komprimiert zu werden, wobei der erste Sensor (64, 64', 64") mit der Wölbung (63) verbunden ist, um den Druck darin zu messen.

9. MRI-System, das Folgendes umfasst:
einen Hauptmagneten (10), der dafür vorgesehen ist, ein statisches Magnetfeld im Patienten zu erzeugen;
Gradientenspulen (20), die dafür vorgesehen sind, dem statischen Magnetfeld Gradientenmagnetfelder zu überlagern;
Hochfrequenzspulen (18), die dafür vorgesehen sind, Hochfrequenzfelder zu induzieren;
eine Steuereinheit (22), die dafür vorgesehen ist, die Gradientenspulen und die Hochfrequenzspulen zu steuern, um Magnetresonanzinformationen von dem Patienten zu erfassen; und
die physiologische Zustandsmessvorrichtung (40, 40', 40") nach einem der Ansprüche 1 bis 8.

10. MRI-System nach Anspruch 9, wobei die Steuereinheit (22) dafür vorgesehen ist, die Ausgabe von der physiologischen Zustandsmessvorrichtung zu empfangen und die Gradienten- und Hochfrequenzspulen so zu steuern, dass während eines vorgewählten physiologischen Zustand des Patienten Magnetresonanzinformationen erfasst werden.

11. MRI-System nach Anspruch 9, das weiterhin Folgendes umfasst:
einen Rekonstruktionsprozessor (26), der dafür vorgesehen ist, die Ausgabe der physiologischen Zustandsmessvorrichtung zu empfangen und Bilder des Patienten in einem oder mehreren ausgewählten physiologischen Zuständen zu rekonstruieren.

12. Verfahren (500) zum Messen eines physiologischen Zustands eines Patienten (4) und Minimieren eines Maßes an Interferenz, die während eines Magnetresonanz- (MR) Scans erzeugt wird, wobei das Verfahren Folgendes umfasst:
mit einem ersten aktiven Sensor (64, 64', 64") Messen des physiologischen Zustands und der MR-Scan-bezogene Interferenz und Erzeugen eines ersten Signals (80, 80', 80") mit einer physiologischen Zustandskomponente (76) und einer Interferenzkomponente (78);
mit einem zweiten nicht-aktiven Sensor (70), der eng angrenzend an den ersten Sensor (64, 64', 64") angeordnet ist, so dass er im Wesentlichen die gleiche MR-bezogene Interferenz erfährt wie der erste Sensor (64, 64', 64"), Messen der MR-Scanbezogenen Interferenz und Erzeugen eines zweiten Signals (82) mit der Interferenzkomponente; und
subtraktives Kombinieren der ersten und zweiten Signale, um die Interferenzkomponente (78) aufzuheben und ein Signal mit der physiologischen Zustandskomponente zu erzeugen;
wobei der erste aktive Sensor einen ersten Druckwandler umfasst, der dafür vorgesehen ist, einen Druck in Zusammenhang mit dem physiologischen Zustand zu messen, und wobei der zweite nicht-aktive Sensor einen zweiten Druckwandler umfasst, der so vorgesehen ist, dass er daran gehindert wird, Druck zu messen.

13. Verfahren nach Anspruch 12, wobei der gemessene physiologische Zustand in einem vordefinierten Frequenzbereich variiert und weiterhin Folgendes umfassend:
Filtern der ersten und zweiten Signale, um Frequenzkomponenten über dem vordefinierten Frequenzbereich zu entfernen.

14. Verfahren zum Betreiben eines MRI-Systems, umfassend:
Erzeugen statischer Magnetfelder in einem Patienten;
Überlagern der statischen Magnetfelder mit Gradientenmagnetfeldern;
Überlagern von Hochfrequenzfeldern, um magnetische Resonanz in den Patienten zu induzieren;
Erfassen von magnetischen Resonanzinformationen von dem Patienten;
Messen eines physiologischen Zustands des Patienten mit dem Verfahren nach Anspruch 12 oder Anspruch 13.

15. MRI-Verfahren nach Anspruch 14, weiterhin umfassend eines von Folgendem:
Verwenden der physiologischen Zustandskomponente zum Steuern der Überlagerung der Gradientenmagnetfelder und der Hochfrequenzfelder, um die Magnetresonanzinformation nur dann zu erfassen, wenn sich der Patient in einem ausgewählten physiologischen Zustand befindet; oder
Rekonstruieren der erfassten Magnetresonanzinformationen zu Bildern und Verwenden der physiologischen Zustandskomponente, um das Rekonstruieren so zu steuern, dass die Bilder anhand der Magnetresonanzinformationen rekonstruiert werden, wenn der Patient einen oder mehrere ausgewählte physiologische Zustände hatte.

## Revendications

1. Dispositif de détection de statut physiologique (40, 40', 40") pour détecter un statut physiologique d'un patient (4) et minimiser une quantité d'interférence (78) générée pendant un balayage de résonance magnétique (RM) par un système à résonance magnétique (RM) (8), le dispositif (40) comprenant :
un premier capteur actif (64, 64', 64") situé de façon à pouvoir détecter le statut physiologique et subir une interférence liée au balayage RM pendant un balayage RM, et conçu pour fournir en sortie un premier signal (80, 80', 80") pendant le balayage ayant une composante de statut physiologique (76) et une composante d'interférence (78) ;
un second capteur non actif (70, 70', 70") situé étroitement adjacent au premier capteur (64, 64', 64") de façon à subir sensiblement la même interférence liée au balayage RM que le premier capteur (64, 64', 64") pendant un balayage RM, et conçu pour fournir en sortie un second signal (82, 82', 82") pendant le balayage ayant la composante d'interférence (78); et
un circuit (50, 84, 110, 110', 116, 116') qui opère de façon soustractive sur les premier (80, 80', 80") et second (82) signaux pour annuler la composante d'interférence (78)
dans lequel le premier capteur actif comporte un premier transducteur de pression conçu pour détecter une pression liée au statut physiologique et le second capteur non actif comporte un second transducteur de pression conçu de façon à être empêché de détecter une pression.

2. Dispositif (40, 40', 40") selon la revendication 1, dans lequel les premier et second transducteurs de pression comprennent des transducteurs de pression piézorésistifs de construction similaire.

3. Dispositif (40, 40', 40") selon l'une ou l'autre des revendications 1 et 2, dans lequel les premier (64, 64', 64") et second (70, 70', 70") capteurs ont une construction similaire et sont montés avec une orientation commune sur un substrat commun.

4. Dispositif (40, 40', 40") selon l'une quelconque des revendications 1 à 3, comprenant en outre :
une unité de filtre d'interférence (110, 110', 110") qui est couplée électriquement au premier signal (80, 80', 80") ayant une première sortie différentielle (80, 80', 80") et au second signal (82, 82', 82") ayant une deuxième sortie différentielle (82, 82', 82") du premier capteur (64, 64', 64") et du second capteur (70, 70', 70") respectivement, et qui est conçue pour éliminer la composante d'interférence (78) de la première (80, 80', 80") et de la deuxième (82, 82', 82") sortie différentielle, et pour transmettre la composante de statut physiologique (76), dans lequel la première (80, 80', 80") et la deuxième (82, 82', 82") sortie différentielle ont chacune des signaux de sortie complémentaires transmis à leur niveau.

5. Dispositif (40, 40', 40") selon l'une quelconque des revendications 1 à 3, dans lequel le circuit (110, 110', 110") comporte :
un premier amplificateur (91, 132) couplé au premier capteur (64, 64', 64") et ayant une troisième sortie (112) ;
un second amplificateur (93, 134) couplé au second capteur (70, 70', 70") et ayant une quatrième sortie (114) ;
un amplificateur différentiel (116, 116', 116") conçu pour combiner de façon soustractive les troisième et quatrième sorties afin de générer un signal de pression ; et
un convertisseur (124, 124', 124") conçu pour numériser le signal de pression.

6. Dispositif selon l'une quelconque des revendications 1 à 5, dans lequel au moins l'un du capteur actif (64") et du capteur non actif (70") est dans un pont de Wheatstone et comportant en outre :
un étage de pilote actif (126) couplé à une borne de référence (144) du premier capteur actif (64") et comportant :
des circuits tampons à impédance élevée (130) couplés au premier capteur (64") et au second capteur non actif (70") pour fournir une sortie à impédance élevée (136) au niveau de chaque sortie de capteur (80", 82") ;
un réseau de moyennage de résistor (138) conçu pour moyenner chaque sortie à impédance élevée (136) des circuits tampons à impédance élevée (130) et pour générer un signal moyenné à partir de ceux-ci ; et
un dispositif de gain (β) couplé à un inverseur (140) conçu pour inverser le signal moyenné et pour polariser le pont de Wheatstone avec le signal moyenné inversé.

7. Dispositif (40, 40', 40") selon l'une quelconque des revendications 1 à 6, dans lequel le statut physiologique est un état et une fonction respiratoires, le dispositif comportant en outre :
une ceinture ou sangle (44) configurée pour encercler la taille du patient ;
une poche (61) montée sur la ceinture (44) et conçue pour être comprimée pendant le cycle respiratoire du patient, dans lequel le premier capteur (64, 64', 64") est connecté à la poche pour y détecter une pression.

8. Dispositif (40, 40', 40") selon l'une quelconque des revendications 1 à 6, dans lequel le statut physiologique est un état de pression artérielle, le dispositif comportant en outre :
un dôme (63) monté pour être comprimé avec des changements de pression artérielle, dans lequel le premier capteur (64, 64', 64") est connecté au dôme (63) pour y détecter une pression.

9. Système d'IRM comprenant :
un aimant principal (10) conçu pour générer un champ magnétique statique chez un patient ;
des bobines de gradient (20) conçues pour imposer des champs magnétiques de gradient sur le champ magnétique statique ;
des bobines de radiofréquence (18) conçues pour induire des champs de radiofréquence ;
un dispositif de commande (22) conçu pour commander les bobines de gradient et les bobines de radiofréquence afin d'acquérir des informations de résonance magnétique du patient ; et
le dispositif de détection de statut physiologique (40, 40', 40") selon l'une quelconque des revendications 1 à 8.

10. Système d'IRM selon la revendication 9, dans lequel le dispositif de commande (22) est conçu pour recevoir la sortie du dispositif de détection de statut physiologique et pour commander les bobines de gradient et de radiofréquence afin d'acquérir des informations de résonance magnétique pendant un statut physiologique présélectionné du patient.

11. Système d'IRM selon la revendication 9, comportant en outre :
un processeur de reconstruction (26) configuré pour recevoir la sortie du dispositif de détection de statut physiologique et pour reconstruire des images du patient dans un ou plusieurs statuts physiologiques sélectionnés.

12. Procédé (500) de détection d'un statut physiologique d'un patient (4) et de minimisation d'une quantité d'interférence générée pendant un balayage de résonance magnétique (RM), le procédé comprenant :
avec un premier capteur actif (64, 64', 64"), la détection d'un statut physiologique et d'une interférence liée au balayage RM et la génération d'un premier signal (80, 80', 80") ayant une composante de statut physiologique (76) et une composante d'interférence (78) ;
avec un second capteur non actif (70) positionné étroitement adjacent au premier capteur (64, 64', 64") de façon à subir sensiblement la même interférence liée au balayage RM que le premier capteur (64, 64', 64"), la détection de l'interférence liée au balayage RM et la génération d'un second signal (82) ayant la composante d'interférence ; et
la combinaison de façon soustractive des premier et second signaux pour annuler la composante d'interférence (78) et générer un signal avec la composante de statut physiologique ;
dans lequel le premier capteur actif comporte un premier transducteur de pression conçu pour détecter une pression liée au statut physiologique et le second capteur non actif comporte un second transducteur de pression conçu de façon à être empêché de détecter une pression.

13. Procédé selon la revendication 12, dans lequel le statut physiologique détecté varie dans une plage de fréquences prédéfinie et comportant en outre :
le filtrage des premier et second signaux pour éliminer des composantes de fréquence au-dessus de la plage de fréquences prédéfinie.

14. Procédé d'exploitation d'un système d'IRM comprenant :
la génération de champs magnétiques statiques chez un patient ;
le fait d'imposer des champs magnétiques de gradient sur les champs magnétiques statiques ;
le fait d'imposer des champs de radiofréquence pour induire une résonance magnétique chez le patient ;
l'acquisition d'informations de résonance magnétique du patient ;
la détection d'un statut physiologique du patient avec le procédé selon la revendication 12 ou la revendication 13.

15. Procédé d'IRM selon la revendication 14, comportant en outre l'une parmi :
l'utilisation de la composante de statut physiologique pour commander le fait d'imposer les champs magnétiques de gradient et les champs de radiofréquence de façon à acquérir les informations de résonance magnétique uniquement lorsque le patient a un statut physiologique sélectionné ; ou
la reconstruction en images des informations de résonance magnétique acquises et l'utilisation de la composante de statut physiologique pour commander la reconstruction de façon à reconstruire les images à partir d'informations de résonance magnétique lorsque le patient a un ou plusieurs statuts physiologiques sélectionnés.
